# EUROPÄISCHE PATENTANMELDUNG

(11) **EP 1 647 266 A1**
(43) Veröffentlichungstag der Anmeldung: **19.04.2006**
(21) Anmeldenummer: 05019951.2
(22) Anmeldetag: 17.12.2003
(51) Int. Cl.: A61K 8/41, A61Q 5/10

(54) **Neue Kupplerkomponenten**

(30) Priorität: 23.12.2002 DE 10260834
(62) Teilanmeldung aus: 03795902.0
(71) Anmelder: Henkel Kommanditgesellschaft auf Aktien, 40589 Düsseldorf (DE)
(72) Erfinder: Knübel, Georg, 40219 Düsseldorf (DE); Nemitz, Ralph, 41363 Jüchen (DE); Höffkes, Horst, 40595 Düsseldorf (DE)

(57) **Zusammenfassung**

Gegenstand der vorliegenden Anmeldung sind Mittel zur Färbung keratinischer Fasern, insbesondere menschlicher Haare, enthaltend in einem kosmetisch akzeptablen Träger als Kupplerkomponente mindestens ein m-Phenylendiaminderivat der Formel (I) 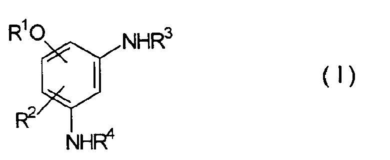 wobei R¹ steht für eine C₁- bis C₄-Alkylgruppe oder eine C₁- bis C₄-Monohydroxyalkylgruppe,
R² steht für ein Wasserstoffatom, eine Methyl- oder eine Ethylgruppe, und
R³ und R⁴ stehen unabhängig voneinander für eine verzweigte oder unverzweigte C₂- bis C₆-Hydroxyalkylgruppe, mit der Maßgabe, dass die Gruppe R¹O in o-Stellung oder m-Stellung zu jeweils beiden Aminogruppen steht. Die erfindungsgemäßen Kupplerkomponenten ermöglichen, insbesondere mit p-Toluylendiamin, 1-(2-Hydroxyethyl)-2,5-diaminobenzol, 2,4,5,6-Tetraaminopyrimidin und Bis-(2-hydroxy-5-aminophenyl)methan, Färbungen mit hohen Farbintensitäten und guten bis sehr guten Echtheitseigenschaften im Rot- und Violettbereich.

## Beschreibung

Die vorliegende Erfindung betrifft Mittel zur Färbung keratinischer Fasern, die spezielle m-Phenylendiaminderivate enthalten, ein Verfahren zur Färbung von Haaren mit diesen Mitteln, sowie einige dieser m-Phenylendiaminderivate selbst und Zwischenprodukte, die bei der Herstellung dieser Verbindungen entstehen.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2-hydroxyethyl)-N,N'-bis(4-aminophenyl)-diaminopropan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin und 2-Methyl-4-chlor-5-aminophenol.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme, Schweiß, Reibung und den Einfluss chemischer Reduktionsmittel, z.B. Dauerwellenflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffkomponenten, die auch in toxikologischer und dermatologischer Hinsicht unproblematisch sind.

Aufgabe der vorliegenden Erfindung war es daher, neue Kupplerkomponenten zu entwickeln, die die an Oxidationsfarbstoffvorprodukte gestellten Anforderungen, insbesondere hinsichtlich der toxikologischen und dermatologischen Eigenschaften, erfüllen und Färbungen in einem breiten Farbspektrum mit guten Echtheitseigenschaften ermöglichen.

Überraschenderweise wurde nun gefunden, dass spezielle m-Phenylendiaminderivate, den an Kupplerkomponenten gestellten Anforderungen in einem hohen Maße genügen. Die erfindungsgemäßen Kupplerkomponenten ermöglichen, insbesondere mit p-Toluylendiamin, 1-(2-Hydroxyethyl)-2,5-diaminobenzol, 2,4,5,6-Tetraaminopyrimidin und Bis-(2-hydroxy-5-aminophenyl)methan, Färbungen mit hohen Farbintensitäten und guten bis sehr guten Echtheitseigenschaften im Rot- und Violettbereich.

Ein erster Gegenstand der vorliegenden Erfindung sind daher Mittel zur Färbung keratinischer Fasern, insbesondere menschlicher Haare, enthaltend in einem kosmetisch akzeptablen Träger als Kupplerkomponente mindestens ein m-Phenylendiaminderivat der Formel (I) 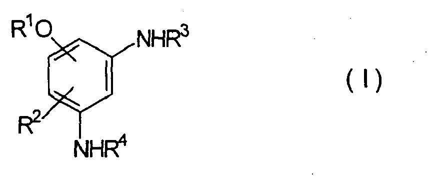 wobei R¹ steht für eine C₁- bis C₄-Alkylgruppe oder eine C₁- bis C₄₋Monohydroxyalkylgruppe,
R² steht für ein Wasserstoffatom, eine Methyl- oder eine Ethylgruppe,
R³ und R⁴ stehen unabhängig voneinander für eine verzweigte oder unverzweigte C₂- bis C₆-Hydroxyalkylgruppe,
mit der Maßgabe, dass die Gruppe R¹O in o-Stellung oder m-Stellung zu jeweils beiden Aminogruppen steht.

Unter keratinischen Fasern sind dabei erfindungsgemäß Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Da es sich bei den erfindungsgemäßen m-Phenylendiaminderivaten um AminoVerbindungen handelt, lassen sich aus diesen in üblicher Weise die bekannten Säureadditionssalze herstellen. Alle Aussagen dieser Schrift und demgemäss der beanspruchte Schutzbereich beziehen sich daher sowohl auf die in freier Form vorliegenden Verbindungen als auch auf deren wasserlösliche, physiologisch verträglichen Salze. Beispiele für solche Salze sind die Hydrochloride, die Hydrobromide, die Sulfate, die Phosphate, die Acetate, die Propionate, die Citrate und die Lactate. Die Hydrochloride und die Sulfate sind dabei besonders bevorzugt.

Beispiele für die als Substituenten in den erfindungsgemäßen Verbindungen genannten C₁- bis C₆-Alkylgruppen sind die Gruppen Methyl, Ethyl, Propyl, Isopropyl, Butyl, Pentyl und Hexyl. Ethyl und Methyl sind bevorzugte Alkylgruppen. Als bevorzugte C₁- bis C₆₋Monohydroxyalkylgruppe können eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt.

Die m-Phenylendiaminderivate der Formel (I) lassen sich mit Hilfe herkömmlicher organischer Methoden herstellen. Beispielhaft sei an dieser Stelle auf die Versuchsdurchführungen im Rahmen der Ausführungsbeispiele verwiesen.

Einige strukturell verwandte m-Phenylendiaminderivate sind bereits aus dem Stande der Technik als Kupplerkomponenten bekannt. In der internationalen Offenlegungsschrift WO-A2-93/10 744 wird ein Verfahren zur Färbung von Haaren in einem sauren Medium (pH < 7) offenbart, bei dem strukturell verwandte m-Phenylendiaminderivate zum Einsatz kommen. Konkret wird dort die Verbindung 2,4-Di-(β-hydroxyethylamino)-1-methoxybenzol beschrieben. Die nunmehr beanspruchten Verbindungen unterscheiden sich von dieser Verbindung, dadurch, dass sie Strukturisomere sind. Dieser Schrift sind keinerlei Hinweise auf die nunmehr beanspruchten Verbindungen und deren hervorragenden färberischen Eigenschaften zu entnehmen.

Erfindungsgemäß können die m-Phenylendiaminderivate der Formel (I) bevorzugt sein, bei denen die Substituenten R³ und R⁴ identisch sind. Besonders bevorzugt stehen die Substituenten R³ und R⁴ jeweils für eine 2-Hydroxyethylgruppe oder eine 3-Hydroxypropylgruppe. Eine 2-Hydroxyethylgruppe ist ganz besonders bevorzugt.

Weiterhin können erfindungsgemäß die m-Phenylendiaminderivate der Formel (I) bevorzugt sein, bei denen der Substituent R¹ für eine C₁- bis C₄-Alkylgruppe steht. Besonders sind die Verbindungen der Formel (I) bevorzugt, bei denen R¹ für eine Methylgruppe steht.

Ferner können erfindungsgemäß die m-Phenylendiaminderivate der Formel (I) bevorzugt sein, bei denen R² für eine Methylgruppe steht.

Je nach Stellung des Substituenten R¹O- im Bezug zu den beiden Aminogruppen lassen sich 2 Gruppen von Isomeren unterscheiden.

Im Rahmen einer ersten Ausführungsform der vorliegenden Erfindung sind die Isomeren bevorzugt, bei denen sich die Gruppe R¹O- in o-Stellung zu den beiden Aminogruppen befindet. Neben den Verbindungen, bei denen R² für Wasserstoff steht, sind die folgenden Verbindungen im Rahmen dieser Ausführungsform umfasst:

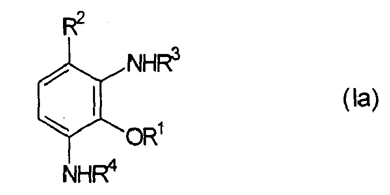

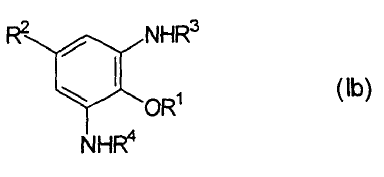

Besonders bevorzugte Verbindungen dieser Ausführungsform sind 2,6-Bis-[(2-hydroxyethyl)amino]-anisol und 2,6-Bis-[(2-hydroxyethyl)amino]-4-methylanisol.

Im Rahmen einer zweiten Ausführungsform der vorliegenden Erfindung sind die Isomeren bevorzugt, bei denen die Gruppe R¹O- sich in m-Stellung zu den beiden Aminogruppen befindet. Im Rahmen dieser Ausführungsform sind die folgenden Verbindungen umfasst:

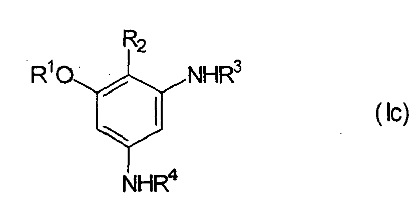

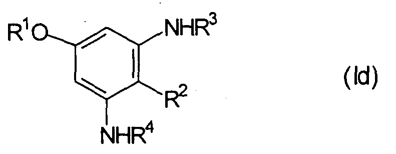

Neben den m-Phenylendiaminderivaten der Formel (I) können die erfindungsgemäßen Färbemittel ferner mindestens eine Entwicklerkomponente enthalten.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 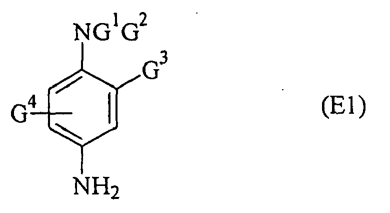 wobei
- G¹ steht für ein Wasserstoffatom, einen C₁- bis C₄-Alkylrest, einen C₁- bis C₄₋Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄₋Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄₋Acetylaminoalkoxyrest, einen C₁- bis C₄- Mesylaminoalkoxyrest oder einen C₁- bis C₄₋Carbamoylaminoalkoxyrest;
- G⁴ steht für ein Wasserstoffatom, ein Halogenatom oder einen C₁- bis C₄₋Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄₋Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄₋Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 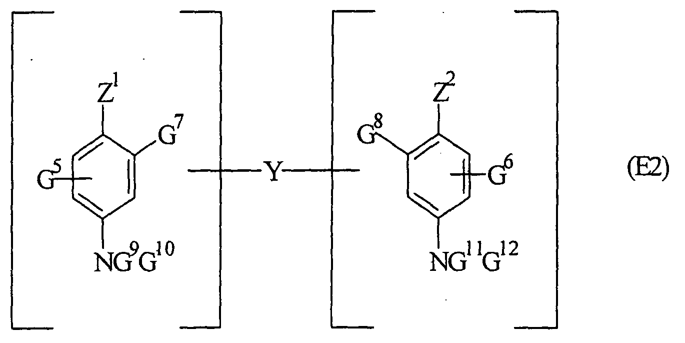 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈₋Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄₋Alkylrest, mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Bis-(2-hydroxy-5-aminophenyl)-methan ist eine ganz besonders bevorzugte zweikernige Entwicklerkomponente der Formel (E2).

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 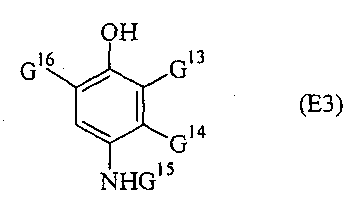 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄₋Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄₋Cyanoalkylrest,
- G¹⁵ steht für Wasserstoff, einen C₁- bis C₄-Alkylrest, einen C₁- bis C₄₋Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 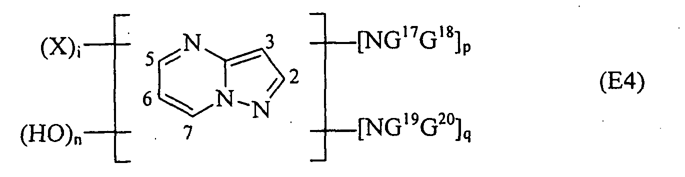 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂₋bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁₋bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁₋bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁₋bis C₄-Alkylrest, einen Aryl-Rest, einen C₁- bis C₄-Hydroxyalkylrest, einen C₂- bis C₄₋Polyhydroxyalkylrest, einen C₁- bis C₄-Aminoalkylrest, einen (C₁- bis C₄)-Alkylamino-(C₁₋bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄₋hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1, mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

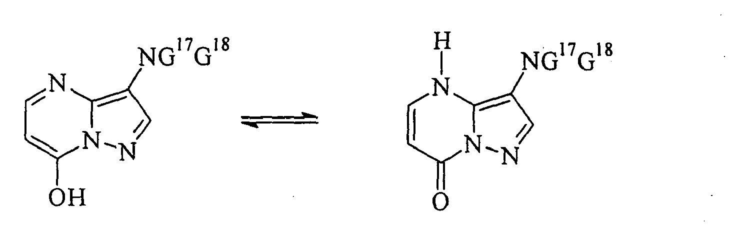

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin; sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform enthalten die erfindungsgemäßen Färbemittel mindestens eine weitere Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypy,rimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als erfindungsgemäß besonders geeignet haben sich die folgenden Kuppler/Entwicklerkombinationen erwiesen:
- 2,6-Bis-[(2-hydroxyethyl)amino]-4-methylanisol / p-Toluylendiamin,
- 2,6-Bis-[(2-hydroxyethyl)amino]-4-methylanisol / 1-(2-Hydroxyethyl-2,5-diaminobenzol,
- 2,6-Bis-[(2-hydroxyethyl)amino]-4-methylanisol / Bis-(2-hydroxy-5-aminophenyl)methan sowie
- 2,6-Bis-[(2-hydroxyethyl)amino]-4-methylanisol / 2,4,5,6-Tetraaminopyrimidin.
   Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.
   In einer weiteren Ausführungsform der vorliegenden Erfindung können die Färbemittel mindestens eine Vorstufe eines naturanalogen Farbstoffs enthalten. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.
   Besonders gut als Vorstufen naturanaloger Haarfarbstoffe geeignet sind Derivate des 5,6-Dihydroxyindolins der Formel (IIa), 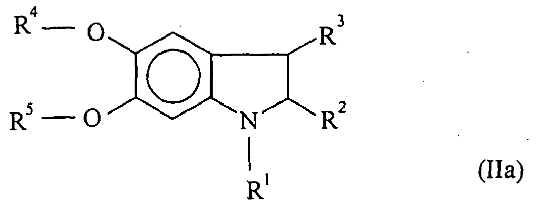 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
   sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbon-säure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIb), 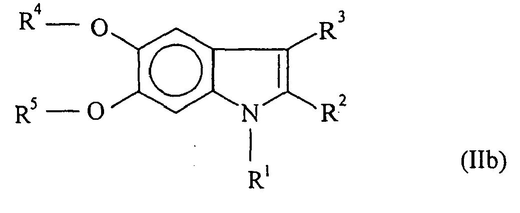 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Färbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Neben den erfindungsgemäßen m-Phenylendiaminderivaten der Formel (I) können die erfindungsgemäßen Färbemittel in einer weiteren bevorzugten Ausführungsform der vorliegenden Erfindung zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

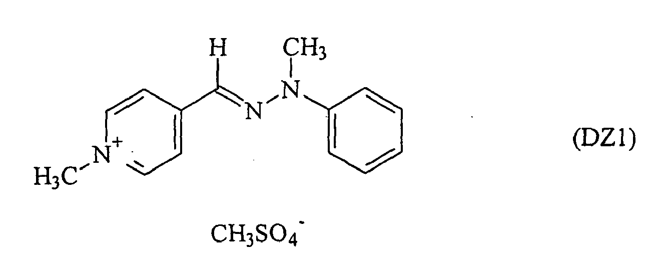

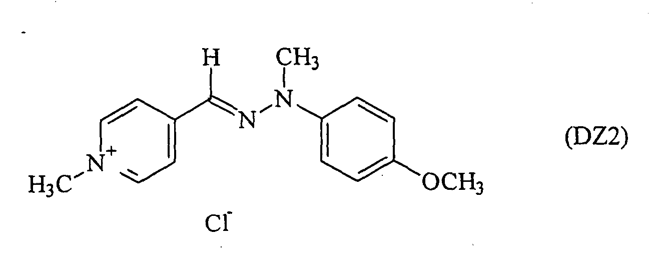

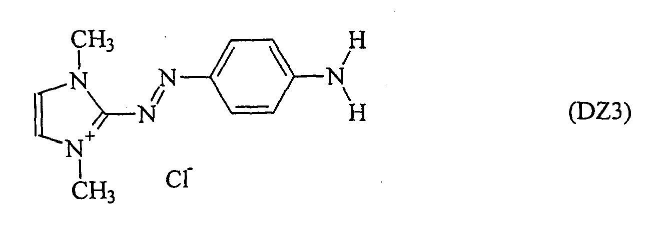

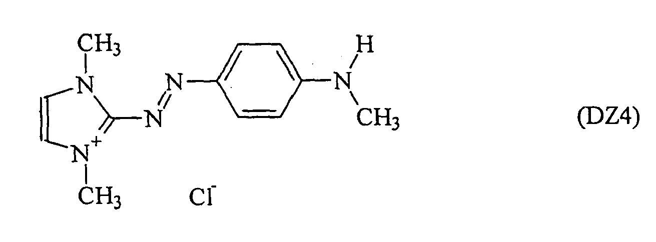

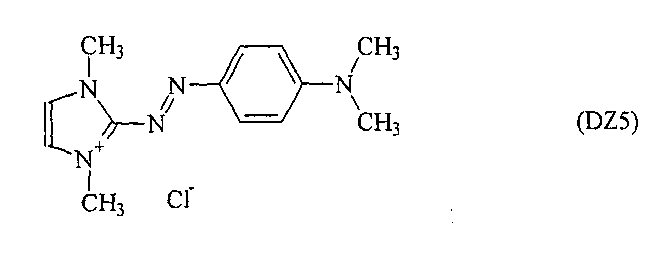

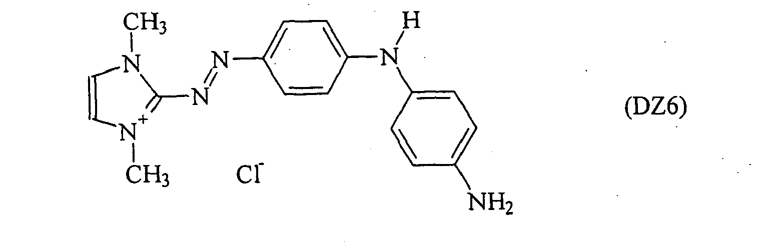

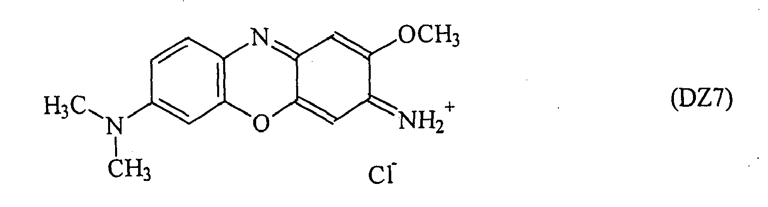

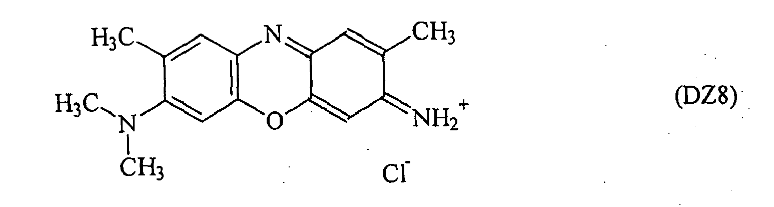

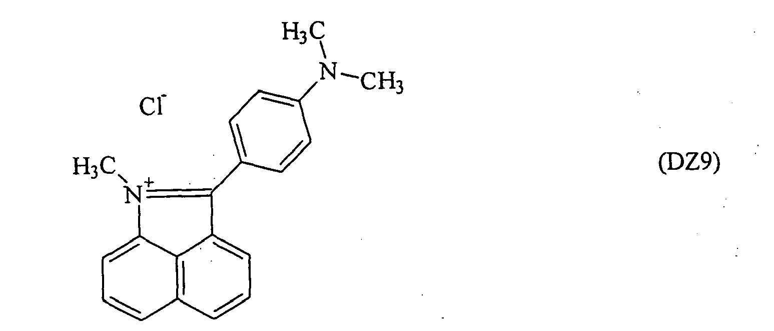

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhätt. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen Färbemittel weitere Wirk-, Hilfs- und Zusatzstoffe, wie beispielsweise
- nichtionische Polymere wie beispielsweise VinylpyrrolidonNinylacrylat-Copolymere, Polyvinylpyrrolidon und VinylpyrrolidonNinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel enthalten die Farbstoffvorprodukte bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-förmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol beziehungsweise Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein zweiter Gegenstand der vorliegenden Anmeldung ist die Verwendung der erfindungsgemäßen m-Phenylendiaminderivate zum Färben keratinischer Fasern.

Ein dritter Gegenstand der vorliegenden Erfindung ist ein Verfahren zur Färbung keratinischer Fasern, bei dem ein erfindungsgemäßes Haarfärbemittel auf die Fasern aufgetragen wird und nach einer Einwirkzeit wieder abgespült wird.

Ein vierter Gegenstand der vorliegenden Erfindung sind m-Phenylendiaminderivate der Formel (I) 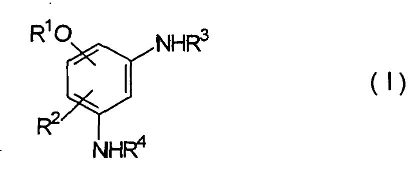 wobei R¹ steht für eine C₁- bis C₄-Alkylgruppe oder eine C₁- bis C₄₋Monohydroxyalkylgruppe,
R² steht für ein Wasserstoffatom, eine Methyl- oder eine Ethylgruppe,
R³ und R⁴ stehen unabhängig voneinander für eine verzweigte oder unverzweigte C₂- bis C₆-Hydroxyalkylgruppe,
mit der Maßgabe, dass die Gruppe R¹O in o-Stellung oder m-Stellung zu jeweils beiden Aminogruppen steht.
m-Phenylendiaminderivate, die ausgewählt sind aus der Gruppe, die gebildet wird von 2,6-Bis-[(2-hydroxyethyl)amino]-4-methylanisol und 2,6-Bis-[(2-hydroxyethyl)amino]-anisol, sind erfindungsgemäß besonders bevorzugt.

Ein fünfter Gegenstand der vorliegenden Erfindung sind die ersten Zwischenstufen der Synthese der erfindungsgemäßen m-Phenylendiamine, ausgewählt aus der Gruppe, die gebildet wird von Bis(2-chlorethyl) (2-methoxy-5-methyl-1,3-phenylen)biscarbamat und Bis(2-chlorethyl)(2-methoxy-1,3-phenylen)biscarbamat.

Ein sechster Gegenstand der vorliegenden Erfindung sind die zweiten Zwischenstufen der Synthese der erfindungsgemäßen m-Phenylendiamine, ausgewählt aus der Gruppe, die gebildet wird von 3,3'-(2-Methoxy-5-methyl-1,3-phenylen)bis(1,3-oxazolidin-2-on) und 3,3'-(2-Methoxy-1,3-phenylen)bis(1,3-oxazolidin-2-on).

### Ausführungsbeispiele

### 1 Synthesen

### 1.1 2,6-Bis-[(2-hydroxyethyl)amino]-4-methylanisol

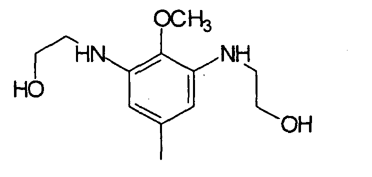

### 1.1.1 2,6-Diamino-4-methylanisol-dihydrochlorid

100 g 2,6-Dinitro-4-methylanisol, 1,35 I Methanol, 150 ml Wasser und 1 g Pd/C (5 %ig) wurden im Autoklav vorgelegt und bei 50 °C mit 50 bar Wasserstoff-Druck 12 h hydriert. Im Anschluss ließ man abkühlen, und der Ansatz wurde anschließend in 1,0 l halbkonzentrierte HCl gegossen. Der Katalysator wurde abfiltriert, das Filtrat am Rotavapor bis zur Trockne eingeengt und über Nacht im Vakuum getrocknet.

| | |
|---|---|
| Ausbeute: | quantitativ |

### 1.1.2 Bis(2-chlorethyl) (2-methoxy-5-methyl-1,3-phenylen)biscarbamat

56 g 2,6-Diamino-4-methylanisol-dihydrochlorid und 134 g Calciumcarbonat wurden in 1l Dioxan vorgelegt und auf 90 °C erwärmt. Innerhalb von 15 min wurden 80 g 2-Chlorethyl-chloroformiat zugegeben, und der Ansatz wurde weitere 4 h bei dieser Temperatur gerührt. Im Anschluss ließ man abkühlen, und die Mineralsalze wurden abfiltriert. Das Filtrat wurde auf Eiswasser gegossen, die entstandenen hellbraunen Kristalle wurden abfiltriert und im Vakuum getrocknet.

| | |
|---|---|
| Ausbeute: | 50,4 g (63 %) |
| Schmelzpunkt: | 116 - 118 °C |

### 1.1.3 3,3'-(2-Methoxy-5-methyl-1,3-phenylen)bis(1,3-oxazolidin-2-on)

200 ml Natronlauge (20%ig) wurden vorgelegt und auf 45 °C erwärmt. Im Anschluss wurden 50 g Bis(2-chlorethyl) (2-methoxy-5-methyl-1,3-phenylen)biscarbamat portionsweise hinzugegeben, der Ansatz wurde mit 200 ml Dioxan verdünnt und weitere 2 h bei 45 °C nachgerührt. Nach Rühren über Nacht bei Raumtemperatur wurde der Ansatz auf Eis gegossen und mit Salzsäure neutralisiert. Das ausgefallene Produkt wurde abgesaugt und über Nacht im Vakuum getrocknet.

| | |
|---|---|
| Ausbeute: | 24 g (59 %) |
| Schmelzpunkt: | 173 °C |

### 1.1.4 2,6-Bis-[(2-hydroxyethyl)amino]-4-methylanisol

33,5 g 3,3'-(2-Methoxy-5-methyl-1,3-phenylen)bis(1,3-oxazolidin-2-on) wurden in 500 ml 20 %iger KOH 10 h unter Rückfluss erwärmt. Man ließ auf Raumtemperatur abkühlen, und die ausgefallenen Rückstände wurden abfiltriert. Das Filtrat wurde mit 200 ml Ethanol verdünnt und mit konzentrierter HCl auf pH 8 eingestellt. Das ausgefallene KCI wurde abfiltriert und das Filtrat am Rotavapor eingeengt.

| | |
|---|---|
| Ausbeute: | quantitativ |
| Schmelzpunkt: | 82 - 84 °C |

### 1.2 2,6-Bis-[(2-hydroxyethyl)amino]-anisol

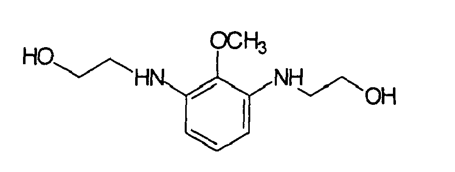

### 1.2.1 4-Chlor-2,6-dinitroanisol

100 ml rauchende Salpetersäure wurden bei 5 - 10 °C vorgelegt, und 50 g 4-Chloranisol wurden bei dieser Temperatur langsam zugetropft. Anschließend erfolgte die tropfenweise Zugabe von 50 ml konzentrierter Schwefelsäure und weiteren 50 ml rauchender Salpetersäure. Es wurde 15 min nachgerührt, und der Ansatz dann auf 500 ml Eis gegeben. Das ausgefallene Produkt wurde abgesaugt, neutral gewaschen und im Vakuum getrocknet.

| | |
|---|---|
| Ausbeute: | 71,7 g (88 %) |
| Schmelzpunkt: | 61 - 62 °C |

### 1.2.2 2,6-Diaminoanisol

23,3 g 2,6-Dinitro-4-chloranisol, 450 ml Ethanol, 50 ml Wasser, 9,8 g Kaliumacetat und 0,1 g Pd/C (5 %ig) wurden im Autoklav vorgelegt und bei 50 °C mit 50 bar Wasserstoff-Druck 12 h hydriert. Im Anschluss ließ man abkühlen, der Katalysator wurde abfiltriert, und das Filtrat wurde am Rotavapor bis zur Trockne eingeengt. Abschließend wurde im Kugelrohr destilliert.

| | |
|---|---|
| Ausbeute: | 4,1 g (29 %) |

### 1.2.3 Bis(2-chlorethyl)(2-methoxy-1,3-phenylen)biscarbamat

14 g 2,6-Diaminoanisol und 18,9 g Calciumcarbonat wurden in 0,5 I Dioxan vorgelegt und auf 90 °C erwärmt. Innerhalb von 15 min wurden 35,8 g 2-Chlorethyl-chloroformiat zugegeben, und es wurde weitere 4 h bei dieser Temperatur gerührt. Im Anschluss ließ man abkühlen, und die Mineralsalze wurden abfiltriert. Das Filtrat wurde auf Eiswasser gegossen, die gebildeten hellbraune Kristalle abfiltriert und im Vakuum getrocknet.

| | |
|---|---|
| Ausbeute: | 19,6 g (56 %) |
| Schmelzpunkt: | 135 - 137 °C |

### 1.2.4 3,3'-(2-Methoxy-1,3-phenylen)bis(1,3-oxazolidin-2-on)

100 ml Natronlauge (20%ig) wurden vorgelegt und auf 45 °C erwärmt. Im Anschluss wurden 19 g Bis(2-chlorethyl) (2-methoxy-1,3-phenylen)biscarbamat portionsweise hinzugegeben, der Ansatz mit 100 ml Dioxan verdünnt und weitere 2 h bei 45 °C nachgerührt. Nach Rühren über Nacht bei Raumtemperatur wurde der Ansatz auf Eis gegossen und mit Salzsäure neutralisiert. Es wurde mehrfach mit Essigsäureethylester extrahiert, und die vereinigten organischen Phasen wurden im Vakuum bis zur Trockne eingeengt.

| | |
|---|---|
| Ausbeute: | 6,2 g (41 %) |

### 1.2.5 2,6-Bis-[(2-hydroxyethyl)amino]-anisol

6,2 g 3,3'-(2-Methoxy-1,3-phenylen)bis(1,3-oxazolidin-2-on) wurden in 100 ml 20 %iger KOH 10 h unter Rückfluss erwärmt. Man ließ auf Raumtemperatur abkühlen, und ausgefallenes KCI wurde abfiltriert. Das Filtrat wurde mit 200 ml Ethanol verdünnt und mit konzentrierter HCl auf pH 8 eingestellt. Das ausgefallene KCI wurde abfiltriert und das Filtrat am Rotavapor eingeengt. Abschließend wurde im Kugelrohr destilliert.

| | |
|---|---|
| Ausbeute: | 3,7 g (73 %) |
| Schmelzpunkt: | 82 - 84 °C |

### 2 Ausfärbungen

### 2.1 Versuchsdurchführung

Für die Herstellung der Färbecreme wurden 50g einer Cremebasis in einem 250ml Becherglas eingewogen und bei 80°C geschmolzen. Die verwendete Cremebasis hatte die folgende Zusammensetzung:

| | |
|---|---|
| Hydrenol® D¹ | 17,0 Gew.-% |
| Lorol® tech.² | 4,0 Gew.-% |
| Texapon® NSO³ | 40,0 Gew.-% |
| Dehyton® K⁴ | 25,0 Gew.-% |
| Eumulgin® B2⁵ | 1,5 Gew.-% |
| Wasser | 12,5 Gew.-% |

| | |
|---|---|
| ¹ C₁₆₋₁₈-Fettalkohol (INCI-Bezeichnung: Cetearyl alcohol) (Cognis) | |
| ² C₁₂₋₁₈-Fettalkohol (INCI-Bezeichnung: Coconut alcohol) (Cognis) | |
| ³ Laurylethersulfat, Natriumsalz (ca. 27,5% Aktivsubstanz; INCI-Bezeichnung: Sodium Laureth Sulfate) (Cognis) | |
| ⁴ N,N-Dimethyl-N-(C₈₋₁₈-kokosamidopropyl)ammoniumacetobetain (ca. 30% Aktivsubstanz; INCI-Bezeichnung: Aqua (Water), Cocamidopropyl Betaine) (Cognis) | |
| ⁵ Cetylstearylalkohol mit ca. 20 EO-Einheiten (INCI-Bezeichnung: Ceteareth-20) (Cognis) | |

Es wurden jeweils 1/400 Mol der Entwickler- bzw. Kupplerkomponente getrennt in destilliertem Wasser suspendiert bzw. unter Erwärmen gelöst. Anschließend wurde Ammoniak (<1 ml; 25%ige Ammoniaklösung) zugegeben bis der pH-Wert zwischen 9 und 10 lag.

Die gelösten Farbstoffvorprodukte wurden nacheinander in die heiße Creme eingearbeitet. Anschließend wurde mit destilliertem Wasser auf 97g aufgefüllt und mit Ammoniak ein pH-Wert von 9,5 eingestellt. Nach Auffüllen mit destilliertem Wasser auf 100 g wurde der Ansatz kaltgerührt (< 30°C), wobei eine homogene Creme entstand.

Für die Ausfärbungen wurden (soweit nichts anderes vermerkt ist) jeweils 25 g Färbecreme mit 25 g der folgenden Oxidationsmittelzubereitung vermischt.

| | |
|---|---|
| Dipicolinsäure | 0,1 Gew.-% |
| Natriumpyrophosphat | 0,03 Gew.-% |
| Turpinal® SL⁶ | 1,50 Gew.-% |
| Texapon® N28⁷ | 2,00 Gew.-% |
| Acrysol® 22⁸ | 0,60 Gew.-% |
| Wasserstoffperoxid, 50 %ig | 12,00 Gew.-% |
| Natronlauge, 45%ig | 0,80 Gew.-% |
| Wasser | ad 100 Gew.-% |

| | |
|---|---|
| ⁶ 1-Hydroxyethan-1,1-diphosphonsäure (ca. 58 - 61% Aktivsubstanzgehalt; INCI-Bezeichnung: Etidronic Acid, Aqua (Water)) (Solutia) | |
| ⁷ Laurylethersulfat-Natrium-Salz (mind. 26,5 % Aktivsubstanzgehalt; INCI-Bezeichnung: Sodium Laureth Sulfate) (Cognis) | |
| ⁸ Acrylpolymer (ca. 29.5 - 30.5% Festkörper in Wasser; INCI-Bezeichnung: Acrylates/Steareth-20 Methacrylate Copolyme) | |

In jede der so erhaltenen Mischungen wurde eine Haarsträhne (80 % ergraut; 330 mg bis 370 mg schwer) gegeben. Anschließend wurden die Mischungen und die Haarsträhnen auf jeweils ein Uhrglas gegeben und die Haarsträhnen in die Färbecremes gut eingebettet. Nach 30 Minuten (±1 Minute) Einwirkzeit bei Raumtemperatur wurden die Haarsträhnen entnommen und mit einer wässrigen Texapon® EVR-Lösung⁹
⁹ Laurylethersulfat-Natrium-Salz mit speziellen Zusätzen (ca. 34 bis 37% Aktivsubstanzgehalt; INCI-Bezeichnung: Sodium Lauryl Sulfate, Sodium Laureth Sulfate, Lauramide MIPA, Cocamide MEA, Glycol Stearate, Laureth-10) (Cognis) so oft gewaschen, bis der Farbüberschuß entfernt war. Die Haarsträhnen wurden an der Luft getrocknet und ihr Farbton unter der Tageslichtlampe (Farbprüfgerät HE240A) bestimmt und notiert (Taschenlexikon der Farben, A. Kornerup u. J.H. Wanscher, 3. unveränderte Auflage 1981, MUSTER-SCHMIDT Verlag; Zürich, Göttingen).

Die bei den Ausfärbungs-Untersuchungen erhaltenen Ergebnisse sind in den nachstehenden Tabellen aufgeführt.

### 2.2 Ausfärbungen mit 2,6-Bis-[(2-hydroxyethyl)amino]-4-methylanisol

| Entwicklerkomponente | Ausfärbung |
|---|---|
| p-Toluylendiamin-sulfat | dunkelpurpur |
| 2,4,5,6-Tetraaminopyrimidin-sulfat | fraise |
| p-Aminophenol | cerise |
| 4,5-Diamino-1-(2-hydroxyethyl)pyrazol-sulfat | dunkelpurpur |
| 2-(2, 5-Diaminophenyl)ethanol-sulfat | tiefviolett |
| 4-Amino-3-methylphenol | mattrot |
| 4-Amino-2-[(5-amino-2-hydroxyphenyl)methyl]phenoldihydrochlorid | graumagenta |

### 2.3 Ausfärbungen mit 2,6-Bis-[(2-hydroxyethyl)amino]-anisol

| Entwicklerkomponente | Ausfärbung |
|---|---|
| p-Toluylendiamin-sulfat | tiefmagenta |
| 2,4,5,6-Tetraaminopyrimidin-sulfat | braunrot |
| p-Aminophenol | braun |
| 4, 5-Diamino-1-(2-hydroxyethyl) pyrazol-sulfat | tiefviolett |
| 2-(2,5-Diaminophenyl)ethanol-sulfat | dunkelviolett |
| 4-Amino-3-methylphenol | dunkelblond |
| 4-Amino-2-[(5-amino-2-hydroxyphenyl)methyl]phenoldihydrochlorid | violettbraun |

### 3 Weitere Ausfärbungen

### 3.1 Rezeptur 1

| Rohstoff | Gew.-% |
|---|---|
| Fettalkoholgemisch C₁₀-C₂₂ | 6,0 |
| Stenol® 1618¹⁰ | 3,5 |
| Kokoslorol® ¹¹ | 1,5 |
| Behenylalkohol | 1,0 |
| Eumulgin® B 1¹² | 0,3 |
| Eumulgin® B 2 | 0,3 |
| Texapon® NSO | 10,0 |
| Dehyton® K | 5,0 |
| Polymer JR® 400¹³ | 0,3 |
| Gafquat® 755¹⁴ | 0,3 |
| Celquat® L 200¹⁵ | 0,1 |
| Ascorbinsäure | 0,2 |
| Natriummetabisulfit | 0,3 |
| Ammoniumsulfat | 0,4 |
| Hydroxyethandiphosphonsäure | 0,2 |
| Wasserglaslösung, 40 % | 0,5 |
| Parfümöl | 0,3 |
| 1,10-Bis-(2,5-diaminophenyl)-1,4,7,10-tetraoxadecantetrahydrochlorid | 0,05 |
| p-Toluylendiamin-sulfat | 0,22 |
| N, N-Bis-(2'-hydroxyethyl)-p-phenylendiamin-sulfat | 0,15 |
| 2-(2'-Hydroxyethyl)-p-phenylendiamin-sulfat | 0,24 |
| Bis-(5-amino-2-hydroxyphenyl)methan-dihydrochlorid | 0,17 |
| 2,4-Bis-(2'-hydroxyethyl) amino-6-methylanisol | 0,50 |
| 2,6-Bis-[(2'-hydroxyethyl)] amino-4-methylanisol | 0,05 |
| Ammoniak, 25%ig | ad pH 10 |
| Wasser | ad 100 |

| | |
|---|---|
| ¹⁰ C₁₆₋₁₈-Fettalkohol (INCI-Bezeichnung: Cetearyl Alcohol) (Cognis) | |
| ¹¹ C₁₂₋₁₈-Fettalkohol (INCI-Bezeichnung: Coconut Alcohol) (Cognis) | |
| ¹² Cetylstearylalkohol mit ca. 12-EO-Einheiten (INCI-Bezeichnung: Ceteareth-12) (Cognis) | |
| ¹³ quaternierte Hydroxyethylcellulose (INCI-Bezeichnung: Polyquaternium-10) (Amerchol) | |
| ¹⁴ Dimethylaminoethylmethacrylat-Vinylpyrrolidon-Copolymer, quaterniert mit Diethylsulfat (ca. 19% Festkörper in Wasser; INCI-Bezeichnung: Polyquaternium-11) (ISP) | |
| ¹⁵ quaterniertes Cellulose-Derivat (INCI-Bezeichnung: Polyquaternium-4) (National Starch) | |

### 3.2 Rezeptur 2

| Rohstoff | Gew.-% |
|---|---|
| Fettalkoholgemisch C₁₀-C₂₂ | 6,0 |
| Eumulgin® B 1 | 0,3 |
| Eumulgin® B 2 | 0,3 |
| Texapon® NSO | 10,0 |
| Dehyton® K | 5,0 |
| Polymer JR® 400 | 0,3 |
| Gafquat® 755 | 0,3 |
| Celquat® L 200 | 0,1 |
| Ascorbinsäure | 0,2 |
| Natriummetabisulfit | 0,3 |
| Ammoniumsulfat | 0,4 |
| Hydroxyethandiphosphonsäure | 0,2 |
| Wasserglaslösung, 40 % | 0,5 |
| Parfümöl | 0,3 |
| 1,3-N,N'-Bis (2'-hydroxyethyl)-N,N'-Bis-(4-aminophenyl)diaminopropan-2-ol-tetrahydrochlorid | 0,1 |
| p-Phenylendiamin-dihydrochlorid | 0,09 |
| p-Toluylendiamin-sulfat | 0,11 |
| 4-Aminophenol | 0,03 |
| 4-Amino-3-methylphenol | 0,01 |
| 4-Amino-2-aminomethylphenol-dihydrochlorid | 0,01 |
| 4-Amino-2-[(diethylamino) methyl] phenol-dihydrochlorid | 0,01 |
| Bis-(5-amino-2-hydroxyphenyl) methan-dihydrochlorid | 0,40 |
| 4,5-Diamino-2-(2'-hydroxyethyl)pyrazol-sulfat | 0,24 |
| 2,4-Bis-(2'-hydroxyethyl) amino-6-methylanisol | 0,11 |
| 2,6-Bis-[(2'-hydroxyethyl)] amino-4-methylanisol | 0,65 |
| Ammoniak, 25%ig | ad pH 10 |
| Wasser | ad 100 |

### 3.3 Rezeptur 3

| Rohstoff | Gew.-% |
|---|---|
| Fettalkoholgemisch C₁₀-C₂₂ | 6,0 |
| Eumulgin® B 1 | 0,3 |
| Eumulgin® B 2 | 0,3 |
| Texapon® NSO | 10,0 |
| Dehyton® K | 5,0 |
| Polymer JR® 400 | 0,3 |
| Gafquat® 755 | 0,3 |
| Celquat® L 200 | 0,1 |
| Ascorbinsäure | 0,2 |
| Natriummetabisulfit | 0,3 |
| Ammoniumsulfat | 0,4 |
| Hydroxyethandiphosphonsäure | 0,2 |
| Wasserglaslösung, 40 % | 0,5 |
| Parfümöl | 0,3 |
| 2,4,5,6-Tetraaminopyrimidin-sulfat | 0,88 |
| 4-Hydroxy-2,5,6-triaminopyrimidin-sulfat | 0,07 |
| 2,4-Bis-(2'-hydroxyethyl) amino-6-methylanisol | 0,34 |
| 2,6-Bis-[(2'-hydroxyethyl)] amino-4-methylanisol | 0,36 |
| Resorcin | 0,02 |
| 2-Methylresorcin | 0,08 |
| 4-Chlorresorcin | 0,01 |
| Resorcinmonomethylether | 0,01 |
| Ammoniak, 25%ig | ad pH 10 |
| Wasser | ad 100 |

### 3.4 Rezeptur 4

| Rohstoff | Gew.-% |
|---|---|
| Fettalkoholgemisch C₁₀-C₂₂ | 10,0 |
| Texapon® K 14 S 70 C¹⁶ | 2,5 |
| Plantaren® 1200 UP¹⁷ | 2,0 |
| Akypo Soft® 45 NV¹⁸ | 12,0 |
| Eutanol® G¹⁹ | 1,0 |
| Eumulgin® B 1 | 0,5 |
| Eumulgin® B 2 | 0,5 |
| Polymer W 37194²⁰ | 2,0 |
| Cosmedia Guar® C 261²¹ | 0,2 |
| Mirapol® A 15²² | 0,5 |
| Ascorbinsäure | 0,2 |
| EDTA-Dinatriumsalz | 0,1 |
| Natriummetabisulfit | 0,3 |
| Ammoniumsulfat | 0,5 |
| Parfümöl | 0,4 |
| Promois® WK²³ | 2,0 |
| Dow Corning® Q2-1401²⁴ | 0,2 |
| Gluadin® WQ²⁵ | 1,0 |
| p-Toluylendiamin-sulfat | 0,55 |
| N, N-Bis-(2'-Hydroxyethyl)-p-phenylendiamin-sulfat | 0,94 |
| 4-Amino-3-methylphenol | 0,03 |
| 2,4,5,6-Tetraaminopyrimidin-sulfat | 1,0 |
| 2,4-Bis-(2'-hydroxyethyl) amino-6-methylanisol | 0,10 |
| 2,6-Bis-[(2'-hydroxyethyl) amino-4-methylanisol | 0,05 |
| Resorcin | 0,11 |
| 2-Methylresorcin | 0,54 |
| 3-Aminophenol | 0,06 |
| 1,3-Bis-(2',4'-diaminophenoxy)propan-tetrahydrochlorid | 0,001 |
| 2-Amino-3-hydroxypyridin | 0,30 |
| 2-Methylamino-3-amino-6-methoxypyridin | 0,001 |
| 2,7-Dihydroxynaphthalin | 0,03 |
| Ammoniak, 25 %ig | ad pH 10 |
| Wasser | ad 100 |

| | |
|---|---|
| ¹⁶ Laurylmyristylethersulfat-Natrium-Salz (ca. 68% bis 73% Aktivsubstanzgehalt'; INCI-Bezeichnung: Sodium Myreth Sulfate) (Cognis) | |
| ¹⁷ C₁₂-C₁₆-Fettalkohol-1.4-glucosid unkonserviert, borfrei ca. 50-53% Aktivsubstanz)(Cognis Corporation (Emery)) | |
| ¹⁸ Laurylalkohol-4.5-EO-Essigsäure-Natrium-Salz (mind. 21% Aktivsubstanzgehalt; INCI-Bezeichnung: Sodium Laureth-6 Carboxylate) (Chem-Y) | |
| ¹⁹ 2-Octyldodecylalkohol (INCI-Bezeichnung: Octyldodecanol) (Cognis) | |
| ²⁰ ca. 20Gew.-% Aktivsubstanzgehalt in Wasser; INCI-Bezeichnung: Acrylamidopropyltrimonium Chloride/Acrylates Copolymer (Stockhausen) | |
| ²¹ Guarhydroxypropyltrimethylammoniumchlorid (mind. 93% Festkörper; INCI-Bezeichnung: Guar Hydroxypropyltrimonium Chloride) (Cognis CorporationCosmedia) | |
| ²² Poly[N-(3-(dimethylammonium)propyl]-N'-[3-ethylenoxyethylendimethylammonium)-propyl]-harnstoff-di-chlorid (ca. 64% Festkörper in Wasser; INCI-Bezeichnung: Polyquaternium-2) (Rhodia) | |
| ²³ Keratinhydrolysat (INCI-Bezeichnung: Aqua (Water), Hydrolyzed Keratin, Methylparaben, Propylparaben) (Seiwa (Interorgana)) | |
| ²⁴ Dimethylcyclosiloxan Dimethylpolysiloxanol Gemisch (ca. 13% Festkörper; INCI-Bezeichnung: Cyclomethicone, Dimethiconol) (Dow Corning) | |
| ²⁵ Weizenproteinhydrolysat (ca. 31-35% Festkörper; INCI-Bezeichnung: Aqua (Water), Laurdimonium Hydroxypropyl Hydrolyzed Wheat Protein, Ethylparaben, Methylparaben) (Cognis) | |

### 3.5 Rezeptur 5

| Rohstoff | Gew.-% |
|---|---|
| Fettalkoholgemisch C₁₀-C₂₂ | 10,0 |
| Texapon® K 14 S 70 C | 2,5 |
| Plantaren® 1200 UP | 2,0 |
| Akypo Soft® 45 NV | 12,0 |
| Eutanol® G | 1,0 |
| Eumulgin® B 1 | 0,5 |
| Eumulgin® B 2 | 0,5 |
| Polymer W 37194 | 2,0 |
| Cosmedia Guar® C 261 | 0,2 |
| Mirapol® A 15 | 0,5 |
| Ascorbinsäure | 0,2 |
| EDTA-Dinatriumsalz | 0,1 |
| Natriummetabisulfit | 0,3 |
| Ammoniumsulfat | 0,5 |
| Parfümöl | 0,4 |
| Promois® WK | 2,0 |
| Dow Corning® Q2-1401 | 0,2 |
| Gluadin® WQ | 1,0 |
| p-Phenylendiamin-dihydrochlorid | 0,10 |
| N,N-Bis-(2'-Hydroxyethyl)-p-phenylendiamin-sulfat | 0,16 |
| 2-(2'-Hydroxyethyl)-p-phenylendiamin-sulfat | 0,34 |
| 4,5-Diamino-2-(2'-hydroxyethyl) pyrazol-sulfat | 0,30 |
| 2,4-Bis-(2'-hydroxyethyl) amino-6-methylanisol | 0,05 |
| 2,6-Bis-[(2'-hydroxyethyl) amino-4-methylanisol | 0,10 |
| Resorcin | 0,09 |
| 5-(2'-Hydroxyethyl) amino-2-methylphenol | 0,07 |
| 3-Amino-2-chlor-6-methylphenol | 0,20 |
| 2,4-Diaminophenoxyethanol-sulfat | 0,01 |
| 1,3-Bis-(2',4'-diaminophenoxy)propan-tetrahydrochlorid | 0,01 |
| 2-Amino-3-hydroxypyridin | 0,09 |
| 3,5-Diamino-2,6-dimethoxypyridin | 0,005 |
| 2,6-Bis-(2'-hydroxyethylamino) toluol | 0,1 |
| 5,6-Dihydroxyindolin-hydrobromid | 0,05 |
| 4-Amino-2-nitro-diphenylamin-2'-carbonsäure | 0,05 |
| Ammoniak, 25 %ig | ad pH 10 |
| Wasser | ad 100 |

### 3.6 Rezeptur 6

| Rohstoff | Gew.-% |
|---|---|
| Fettalkoholgemisch C₁₀-C₂₂ | 10,0 |
| Texapon® K 14 S 70 C | 2,5 |
| Plantaren® 1200 UP | 2,0 |
| Akypo Soft® 45 NV | 12,0 |
| Eutanol® G | 1,0 |
| Eumulgin® B 1 | 0,5 |
| Eumulgin® B 2 | 0,5 |
| Polymer W® 37194 | 2,0 |
| Cosmedia Guar® C 261 | 0,2 |
| Mirapol® A 15 | 0,5 |
| Ascorbinsäure | 0,2 |
| EDTA-Dinatriumsalz | 0,1 |
| Natriummetabisulfit | 0,3 |
| Ammoniumsulfat | 0,5 |
| Parfümöl | 0,4 |
| Promois® WK | 2,0 |
| Dow Corning® Q2-1401 | 0,2 |
| Gluadin® WQ | 1,0 |
| 2-(2'-Hydroxyethyl)-p-phenylendiamin-sulfat | 0,83 |
| 4-Aminophenol | 0,2 |
| 4-Amino-3-methylphenol | 0,01 |
| Bis-(5-amino-2-hydroxyphenyl) methan-dihydrochlorid | 0,10 |
| 2,4,5,6-Tetraaminopyrimidin-sulfat | 1,10 |
| 4-Hydroxy-2,5-6-triaminopyrimidin-sulfat | 0,15 |
| 2,4-Bis-(2'-hydroxyethyl) amino-6-methylanisol | 0,25 |
| 2,6-Bis-[(2'-hydroxyethyl) amino-4-methylanisol | 0,05 |
| Resorcin | 0,1 |
| 2-Methylresorcin | 0,60 |
| 4-Chlorresorcin | 0,03 |
| 3-Aminophenol | 0,004 |
| 5-Amino-2-methylphenol | 0,03 |
| 3-Amino-2-chlor-6-methylphenol | 0,03 |
| 2-Amino-3-hydroxypyridin | 0,24 |
| 2,6-Dihydroxy-3,4-dimethylpyridin | 0,10 |
| 2,7-Dihydroxynaphthalin | 0,02 |
| 1-Phenyl-3-methylpyrazol-5-on | 0,01 |
| Ammoniak, 25 %ig | ad pH 10 |
| Wasser | ad 100 |

### 3.7 Rezeptur 7

| Rohstoff | Gew.-% |
|---|---|
| Stenol® 1618 | 4,5 |
| Kokoslorol® | 2,5 |
| Behenylalkohol | 1,0 |
| Texapon® NSO | 2,0 |
| Dehyton® K | 1,0 |
| Kaliumoleat | 2,0 |
| Kaliumisostearat | 2,0 |
| Kaliumyristat | 1,0 |
| Westvaco® Diacid H240, K-Salz²⁶ | 2,0 |
| Merquat® 550²⁷ | 0,2 |
| Luviquat® FC 370²⁸ | 0,1 |
| Merquat® 280²⁹ | 0,1 |
| Gafquat® HS-100³⁰ | 0,1 |
| Ascorbinsäure | 0,4 |
| Hydroxyethandiphosphonsäure | 0,2 |
| Parfümöl | 0,4 |
| p-Toluylendiamin-sulfat | 0,10 |
| N,N-Bis-(2'-hydroxyethyl)-p-phenylendiamino-sulfat | 0,88 |
| 2-(2'-Hydroxyethyl)-p-phenylendiamin-sulfat | 0,1 |
| 4,5-Diamino-2-(2'-hydroxyethyl)pyrazol-sulfat | 0,72 |
| 2,4-Bis-(2'hydroxyethyl)amino-6-methyanisol | 0,68 |
| 2,6-Bis-[(2'-hydroxyethyl)amino-4-methylanisol | 0,72 |
| Resorcin | 0,02 |
| 2-Methylresorcin | 0,03 |
| 4-Chlorresorcin | 0,02 |
| 5-Amino-2-methylphenol | 0,01 |
| 5-(2'-Hydroxyethyl) amino-2-methylphenol | 0,05 |
| 5-Amino-4-chlor-2-methylphenol | 0,24 |
| 3-Amino-2-chlor-6-methylphenol | 0,07 |
| 1-Naphthol | 0,01 |
| 1,5-Dihydroxynaphthalin | 0,05 |
| 2,6-Bis-(2'hydroxyethylamino) toluol | 0,15 |
| HC Red 1³¹ | 0,05 |
| HC Red BN³² | 0,05 |
| HC Red B 54³³ | 0,05 |
| Basic Red 51³⁴ | 0,05 |
| Ammoniak, 25 %ig | ad pH 10 |
| Wasser | ad 100 |

| | |
|---|---|
| ²⁶ 4-Hexyl-5(6)-carboxy-2-cyclohexen-1-octansäure-Kalium-Salz (ca. 41% Aktivsubstanz in Wasser) (Westvaco Chemicals) | |
| ²⁷ Dimethyldiallylammoniumchlorid Acrylamid Copolymer (ca. 8.1-9.1% Aktivsubstanz in Wasser; INCI-Bezeichnung: Polyquaternium-7) (Ondeo-Nalco) | |
| ²⁸ Vinylimidazoliummethochlorid-Vinylpyrrolidon-Copolymerisat (30:70) (38-42%Festkörper in Wasser; INCI-Bezeichnung: Polyquaternium-16) (BASF) | |
| ²⁹ Dimethyldiallylammoniumchlorid Acrylsäure Copolymer (ca. 35 Aktivsubstanz in Wasser; INCI-Bezeichnung: Polyquaternium-22) (Ondeo-Nalco) | |
| ³⁰ Vinylpyrrolidon, Methacrylamidopropyltrimethylammoniumchlorid Copolymer (19-21% Aktivsubstanz in Wasser; INCI-Bezeichnung: Polyquaternium-28) (ISP) | |
| ³¹ 4-Amino-2-nitrodiphenylamin | |
| ³² 4[(3-Hydroxypropyl)amino]-3-nitrophenol | |
| ³³ 4-[(2-Hydroxyethyl)amino]-3-nitrophenol (INCI-Bezeichnung: 3-Nitro-p-Hydroxyethylaminophenol) | |
| ³⁴ Azofarbstoff (CIBA) | |

### 3.8 Rezeptur 8

| Rohstoff | Gew.-% |
|---|---|
| Stenol® 1618 | 6,0 |
| Kokoslorol® | 6,0 |
| Eumulgin® B1 | 3,0 |
| Eumulgin® B 2 | 3,0 |
| Eumulgin® RH 40³⁵ | 1,0 |
| Polydiol® 400³⁶ | 5,0 |
| Aminoxyd® WS 35³⁷ | 1,0 |
| EDTA-Dinatriumsalz | 0,1 |
| Natrosol® 250 HHR³⁸ | 1,0 |
| Ascorbinsäure | 0,1 |
| Hydroxyethandiphosphonsäure | 0,2 |
| Parfümöl | 0,3 |
| 2,4,5,6-Tetraaminopyrimidin-sulfat | 1,19 |
| 2,4-Bis-(2'-hydroxyethyl) amino-6-methylanisol | 0,23 |
| 2,6-Bis-[(2'-hydroxyethyl) amino-4-methylanisol | 0,72 |
| 2-Methylresorcin | 0,03 |
| 4-Chlorresorcin | 0,02 |
| 2,6-Bis-(2'hydroxyethylamino) toluol | 0,21 |
| 1,2,3,4-Tetrahydro-6-nitrochinoxalin | 0,15 |
| HC-Yellow 5³⁹ | 0,05 |
| 4-Amino-3-nitrophenol | 0,02 |
| Basic Yellow 87⁴⁰ | 0,05 |
| Basic Orange 31⁴¹ | 0,10 |
| Basic Red 51 | 0,05 |
| Ammoniak, 25%ig | ad pH 10 |
| Wasser | ad 100 |

| | |
|---|---|
| ³⁵ gehärtetes Rizinusöl mit ca. 40-EO-Einheiten (INCI-Bezeichnung: Peg-40 Hydrogenated Castor Oil) (Cognis) | |
| ³⁶ Polyethylenglykol (INCI-Bezeichung: PEG-8) (Cognis) | |
| ³⁷ N,N-Dimethyl-N(C₈₋₁₈-kokosacylamidopropyl)amin-N-oxid (ca. 35% Aktivsubstanzgehalt in Wasser; INCI-Bezeichnung: Cocamidopropylamine Oxide) (Goldschmidt) | |
| ³⁸ Hydroxyethylcellulose (INCI-Bezeichnung: Hydroxyethylcellulose) (Hercules) | |
| ³⁹ N¹-(2-Hydroxyethyl)-4-nitro-1,2-phenylendiamin | |
| ⁴⁰ Methinfarbstoff (CIBA) | |
| ⁴¹ Azofarbstoff (CIBA) | |

Die Rezepturen 1 bis 8 wurden mit der oben beschriebenen Oxidationsmittelzubereitung (Pkt. 2.1) im Verhältnis 1:1 gemischt, und die resultierende Anwendungszubereitung auf Strähnen (Kerling, Naturweiß) aufgetragen. Nach einer Einwirkzeit von 30 Minuten bei Raumtemperatur wurden die Fasern mit Wasser gründlich gespült, mit einem Fön getrocknet und die Ausfärbungen beurteilt. Dabei wurden die folgenden Ergebnisse erhalten:

| Rezeptur-Nummer | Farbresultat |
|---|---|
| 1 | intensives Weinrot |
| 2 | intensives Violett |
| 3 | intensives Orangerot |
| 4 | schwärzliches Rot |
| 5 | dunkles Violett |
| 6 | dunkles Rubin |
| 7 | dunkles Aubergine |
| 8 | leuchtendes Orangerot |

## Patentansprüche

1. Mittel zur Färbung keratinischer Fasern, insbesondere menschlicher Haare, enthaltend in einem kosmetisch akzeptablen Träger als Kupplerkomponente mindestens ein m-Phenylendiaminderivat der Formel (I) 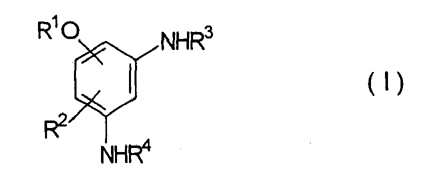 wobei R¹ steht für eine C₁- bis C₄-Alkylgruppe oder eine C₁- bis C₄₋Monohydroxyalkylgruppe,
R² steht für ein Wasserstoffatom, eine Methyl- oder eine Ethylgruppe, und
R³ und R⁴ stehen unabhängig voneinander für eine verzweigte oder unverzweigte C₂- bis C₆-Hydroxyalkylgruppe,
mit der Maßgabe, dass die Gruppe R¹O in o-Stellung oder m-Stellung zu jeweils beiden Aminogruppen steht.

2. Mittel nach Anspruch 1, **dadurch gekennzeichnet, dass** es ein m-Phenylendiaminderivat der Formel (I) enthält, bei dem R³ und R⁴ für identische Substituenten stehen.

3. Mittel nach einem der Ansprüche 1 oder 2, **dadurch gekennzeichnet, dass** es ein m-Phenylendiaminderivat der Formel (I) enthält, bei dem R³ und R⁴ für eine 2-Hydroxyethylgruppe stehen.

4. Mittel nach einem der Ansprüche 1 bis 3, **dadurch gekennzeichnet, dass** es ein m-Phenylendiaminderivat der Formel (I) enthält, bei dem R¹ für eine Methylgruppe steht.

5. Mittel nach einem der Ansprüche 1 bis 4, **dadurch gekennzeichnet, dass** R² steht für eine Methylgruppe.

6. Mittel nach einem der Ansprüche 1 bis 5, **dadurch gekennzeichnet, dass** es ein m-Phenylendiaminderivat der Formel (I) enthält, bei dem sich die Gruppe R¹O- in o-Stellung zu den beiden Aminogruppen befindet.

7. Mittel nach Anspruch 6, **dadurch gekennzeichnet, dass** es ein m-Phenylendiaminderivat der Formel (I) enthält, das ausgewählt ist aus 2,6-Bis-[(2-hydroxyethyl)amino]-anisol und 2,6-Bis-[(2-hydroxyethyl)amino]-4-methylanisol.

8. Mittel nach einem der Ansprüche 1 bis 5, **dadurch gekennzeichnet, dass** es ein m-Phenylendiaminderivat der Formel (I) enthält, bei dem sich die Gruppe R¹O- in m-Stellung zu den beiden Aminogruppen befindet.

9. Mittel nach einem der Ansprüche 1 bis 8, **dadurch gekennzeichnet, dass** es mindestens eine Entwicklerkomponente enthält.

10. Mittel nach einem der Ansprüche 1 bis 9, **dadurch gekennzeichnet, dass** es mindestens eine weitere Kupplerkomponente enthält.

11. Mittel nach einem der Ansprüche 1 bis 10, **dadurch gekennzeichnet, dass** es weiterhin mindestens einen direktziehenden Farbstoff enthält.

12. Mittel nach Anspruch 11, **dadurch gekennzeichnet, dass** der direktziehende Farbstoff kationisch ist.

13. Verfahren zur Färbung keratinischer Fasern, bei dem ein Mittel nach einem der Ansprüche 1 bis 12 auf die Fasern aufgetragen wird und nach einer Einwirkzeit wieder abgespült wird.

14. m-Phenylendiaminderivate der Formel (I) 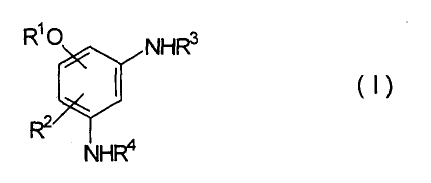 wobei R¹ steht für eine C₁- bis C₄-Alkylgruppe oder eine C₁- bis C₄₋Monohydroxyalkylgruppe,
R² steht für ein Wasserstoffatom, eine Methyl- oder eine Ethylgruppe, und
R³ und R⁴ stehen unabhängig voneinander für eine verzweigte oder unverzweigte C₂- bis C₆-Hydroxyalkylgruppe,
mit der Maßgabe, dass die Gruppe R¹O in o-Stellung oder m-Stellung zu jeweils beiden Aminogruppen steht.

15. Synthesezwischenstufen, ausgewählt aus der Gruppe, die gebildet wird von Bis(2-chlorethyl) (2-methoxy-5-methyl-1,3-phenylen)biscarbamat und Bis(2-chlorethyl)(2-methoxy-1,3-phenylen)biscarbamat.

16. Synthesezwischenstufen ausgewählt aus der Gruppe, die gebildet wird von 3,3'-(2-Methoxy-5-methyl-1,3-phenylen)bis(1,3-oxazolidin-2-on) und 3,3'-(2-Methoxy-1,3-phenylen)bis(1,3-oxazolidin-2-on).
